Europäisches Patentamt

European Patent Office

Office européen des brevets

(11) Veröffentlichungsnummer: **0 260 391**
**A1**

(12) **EUROPÄISCHE PATENTANMELDUNG**

(21) Anmeldenummer: **87109279.7**

(22) Anmeldetag: **27.06.87**

(51) Int. Cl.⁴ **A23K 1/16 , A23K 1/18**

(30) Priorität: **20.08.86 DE 3628248**

(43) Veröffentlichungstag der Anmeldung:
**23.03.88 Patentblatt 88/12**

(84) Benannte Vertragsstaaten:
**AT BE CH ES FR GB IT LI LU NL SE**

(71) Anmelder: **Degussa Aktiengesellschaft**
**Weissfrauenstrasse 9**
**D-6000 Frankfurt am Main 1(DE)**

(72) Erfinder: **Bolze, Rudolf, Dr.**
**Grünaustrasse 11**
**D-6450 Hanau 9(DE)**
Erfinder: **Koch, Friedhelm, Dr.**
**Glatzerstrasse 22**
**D-6451 Grosskrotzenburg(DE)**
Erfinder: **Tanner, Herbert, Dr.**
**Wildaustrasse 20**
**D-6450 Hanau 9(DE)**
Erfinder: **Whitacre, Mark E, Dr.**
**Schanzenstrasse 3**
**D-6450 Hanau 11(DE)**

(54) **Mittel zur Leistungsförderung bei Schweinen und Geflügel.**

(57) Durch Zusatz einer Kombination aus Calcium-oder Magnesiumformiat und Phosphorsäure oder einem sauren Salz der Phosphorsäure zum Futter für junge wachsende Schweine und für Mastgeflügel wird eine höhere bzw. raschere Gewichtszunahme der Tiere und eine bessere Ausnutzung des aufgenommenen Futters erzielt. Bei den Schweinen bewirkt der Zusatz außerdem eine Verminderung des Auftretens von Ferkeldurchfall.

EP 0 260 391 A1

## Mittel zur Leistungsförderung bei Schweinen und Geflügel

Die Erfindung betrifft die Verwendung einer Kombination aus Calcium-oder Magnesiumformiat und Phosphorsäure oder einem sauren Salz der Phosphorsäure als Mittel zur Leistungsförderung bei Schweinen und Geflügel.

Die Leistungsförderung kann sich in einer höheren bzw. rascheren Gewichtszunahme und/oder einer verbesserten Futterverwertung und/oder in verminderten Durchfallerkrankungen zeigen.

Aus in der einschlägigen Literatur veröffentlichten Fütterungsversuchen ist es bekannt, daß der Zusatz verschiedener organischer Säuren zum Futter die Leistung von Nutztieren deutlich verbessern kann.

Eine besonders gute Wirkung haben im allgemeinen Fumarsäure, Propionsäure und Zitronensäure. Diese Säuren können nach ihrer Resorption direkt in den Intermediärstoffwechsel eingreifen und werden dem Zitronensäurezyklus zugeführt und damit für die Energiegewinnung zur Verfügung gestellt.

Zum Teil beruht die Wirksamkeit der dem Futter zugesetzten organischen Säuren auch auf der Beeinflussung der mikrobiellen Besiedlung des Verdauungstraktes durch eine Absenkung des pH-Wertes. Außerdem führt der Zusatz der organischen Säuren zu einer erhöhten Enzymausschüttung über Speichel und Darmwand und zu einer Beeinflussung der bakteriellen Umsetzungen im Darm (M.Kirchgessner und F.X. Roth in "Pig News and Information", Vol. 3, Seiten 259 ff. (1982); A. Halama und E. Roth in "Tierärztl. Umschau" 40, Seiten 105 ff. (1985)).

Organische Säuren werden aufgrund ihrer durch die Absenkung des pH-Wertes bewirkten bakteriostatischen und fungistatischen Eigenschaften außerdem auch zur Konservierung von Futtermitteln oder als Silierhilfsmittel eingesetzt. In der Literatur wird darauf hingewiesen, daß die Wirkung von der undissozierten Säure ausgeht, und daß die entsprechenden Salze hinsichtlich der Geschwindigkeit des Wirkungseintritts, der Wirkungsdauer und der Wirkungsbreite nicht an die Säure heranreichen. Das beruht darauf, daß die Salze nur langsam und unvollständig hydrolysieren (vgl. z.B. Ullmann, Band 19, Seiten 455 ff. (1980)).

Es ist ferner bekannt, daß bei Versuchen zur Leistungsverbesserung durch den Zusatz von Ameisensäure zu Futtermitteln für Broiler nur sehr niedrige Dosierungen einen leicht positiven Effekt auf die Gewichtsentwicklung haben, während bei höheren Dosierungen signifikant schlechtere Gewichtszunahmen beobachtet werden. Die Futterverwertung ist in allen Fällen schlechter als in der Kontrollgruppe ohne Ameisensäure-Zusatz (H.Vogt et al. in "Arch. Geflügelk." 45, Seiten 221 ff. (1981)).

Bei Fütterungsversuchen mit Schweinen führt der Zusatz von 0,6 Gewichtsprozent Ameisensäure zu einer geringfügigen Verbesserung der täglichen Gewichtszunahme, der Zusatz von 1,2 Gewichtsprozent Ameisensäure dagegen zu einer Verringerung. Auch die Futterverwertung ist bei der höheren Dosierung deutlich schlechter (P. Vanderwal in "World's Poultry Sci. J." 35, Seiten 70 ff. (1979)).

Zur ausgewogenen Ernährung von landwirtschaftlichen Nutztieren gehört üblicherweise auch der Einsatz von anorganischen Salzen, wobei vor allem Phosphate von besonderer Wichtigkeit sind. Über den Bedarf hinausgehende Supplementierung mit Mineralstoffen führt allerdings zu negativen Effekten auf die Leistung der Tiere.

Es wurde nun gefunden, daß der Zusatz einer Kombination aus Calcium-oder Magnesiumformiat und Phosphorsäure oder einem sauren Salz der Phosphorsäure zum Futter für junge wachsende Schweine und für Geflügel eine Verbesserung der Gewichtszunahme bewirkt und insbesondere zu einer deutlichen Verbesserung der Futterverwertung führt. Der Zusatz der erfindungsgemäß zu verwendenden Kombination zu einem Futter gegebener Zusammensetzung hat dabei überraschenderweise in allen Fällen eine größere Wirkung als der Zusatz der gleichen Menge an Calcium-oder Magnesiumformiat für sich alleine oder der Zusatz der gleichen Menge an Phosphorsäure oder einem sauren Salz der Phosphorsäure für sich alleine.

Calcium-und Magnesiumformiat sind kristalline Pulver die leicht mit sauren Salzen der Phosphorsäure gemischt werden können. Besonders geeignete saure Salze der Phosphorsäure sind die Natrium-, Kalium-oder Ammoniumsalze, insbesondere das Natrium-oder Ammoniumdihydrogenphosphat. Soll Orthophosphorsäure als solche eingesetzt werden, ist es zweckmäßig, sie an eine feinverteilte Kieselsäure zu binden. So ist beispielsweise eine Mischung aus 65 Gewichtsprozent Phosphorsäure und 35 Gewichtsprozent einer feinteiligen gefällten Kieselsäure frei fließend und gut mit Calcium-oder Magnesiumformiat vermischbar.

Denn es ist zur Vereinfachung einer genauen Dosierung der erfindungsgemäß zu verwendenden Kombination vorteilhaft, sie in Form einer Vormischung aus dem Calcium-oder Magnesiumformiat und der Phosphorsäure oder dem sauren Phosphat einzusetzen. In solchen Vormischungen kann der Anteil des Calcium-oder Magnesiumformiats beispielsweise zwischen 30 und 80 Gewichtsprozent, vorzugsweise zwischen 50 und 60 Gewichtsprozent, liegen.

Ein günstiger Einfluß auf die Gewichtszunahme der Tiere und auf die Futterverwertung wird im allgemeinen mit einer Dosierung einer solchen Vormischung erreicht, die 0,5 bis 3,0 Gewichtsprozent des Futters entspricht.

Die Erfindung soll durch die nachfolgenden Beispiele und Vergleichsversuche verdeutlicht werden:

Beispiel 1:

Es wurde ein Fütterungsversuch mit 96 jungen Schweinen, eingeteilt in 8 Gruppen von jeweils 12 Tieren, unter praxisnahen Bedingungen in Bodenhaltung durchgeführt. Die erste Gruppe erhielt die Normalration, ein Ferkelaufzuchtfutter der folgenden Zusammensetzung (in Gewichtsprozent):

Mais 32
Gerste 25
Tapiokamehl 12
Sojaöl 1
Sojaextraktionsschrot 21
Magermilchpulver 5
Mineralstoffmischung 1,7
Vitamin-und Spurenelementmischung 1,5
Kohlensaurer Kalk 0,8

Die Mischung hatte einen Energiegehalt von 13,5 MJ UE und einen Gehalt von 16,5 Gewichtsprozent Rohprotein. Der Lysingehalt betrug 0,88 Gewichtsprozent. In den weiteren 7 Gruppen wurden erfindungsgemäße Kombinationen aus Calcium-oder Magnesiumformiat und Natriumdihydrogenphosphat mit Fumarsäure und Ameisensäure verglichen, wobei der Anteil des Tapiokamehls um die den eingesetzten Verbindungen entsprechende Menge reduziert wurde.

In der folgenden Tabelle 1 sind für jede der 8 Gruppen die durchschnittliche tägliche Zunahme in Gramm und in %, bezogen auf die Gruppe mit der Normalration (= 100 %), die Futterverwertung in g Futter pro g Zunahme und in %, bezogen wiederum auf die Gruppe mit der Normalration (= 100 %), und die Durchfallhäufigkeit in % zusammengestellt:

Tabelle 1

| Gruppe | | Tägliche Zunahme | | Futterverwertung | | Durchfall- häufigkeit |
|---|---|---|---|---|---|---|
| | | (g) | (%) | (g/g) | (%) | % |
| I | Normalration | 440 | 100 | 2,20 | 100 | 15,6 |
| II | 1,3 % Calcium- formiat + 1,3 % Natrium- dihydrogenphosphat | 505* | 115 | 2,04* | 93 | 5,8 |
| III | 1,3 % Magnesium- formiat + 1,3 % Natriumdi- hydrogenphosphat | 493* | 112 | 2,06* | 94 | 5,1 |
| IV | 1,3 % Calcium- formiat | 480 | 109 | 2,10 | 95 | 6,8 |
| V | 1,3 % Magnesium- formiat | 482 | 110 | 2,09 | 95 | 11,0 |
| VI | 1,3 % Natriumdi- hydrogenphosphat | 439 | 100 | 2,18 | 99 | 12,7 |
| VII | 1,3 % Fumarsäure | 458 | 104 | 2,12 | 96 | 8,0 |
| VIII | 0,9 % Ameisensäure | 468 | 106 | 2,14 | 97 | 6,0 |

* signifikant (p < 0,05)

In dem Versuch wurde durch Kombinationen von Calcium-oder Magnesiumformiat mit sauren Phosphaten eine deutliche Verbesserung der täglichen Zunahme und der Futterverwertung erzielt.

Beispiel 2:

In einem zweiten Versuch wurden entsprechend den Versuchsbedingungen nach Beispiel 1 unterschiedliche Calciumformiat-Dosierungen und Kombinationen aus Calciumformiat und Ammoniumdihydrogenphosphat oder Phosphorsäure kombiniert und die Effekte verglichen.

Die Phosphorsäure wurde in Form einer Kieselsäure-Vormischung zugegeben, wobei 1,3 Gewichtsprozent dieser Vormischung 0,8 Gewichtsprozent Phosphorsäure äquivalent waren.

In der folgenden Tabelle 2 sind wieder für jede der 8 Gruppen die durchschnittliche tägliche Zunahme in Gramm und in %, bezogen auf die Gruppe mit der Normalration ( = 100 %), und die Futterverwertung in g Futter pro g Zunahme und in %, bezogen wiederum auf die Gruppe mit der Normalration ( = 100 %), zusammengestellt:

Tabelle 2

| Gruppe | | Tägliche Zunahme | | Futterverwertung | |
|---|---|---|---|---|---|
| | | (g) | (%) | (g/g) | (%) |
| I | Normalration | 460 | 100 | 2,18 | 100 |
| II | 1 % Calciumformiat + 0,9 % Ammoniumdi- hydrogenphosphat | 497* | 108 | 2,00* | 92 |
| III | 1 % Calciumformiat + 0,8 % Phosphorsäure | 494* | 107 | 2,01* | 92 |
| IV | 1 % Calciumformiat | 482 | 105 | 2,10 | 96 |
| V | 2 % Calciumformiat + 0,9 % Ammoniumdi- hydrogenphosphat | 508* | 110 | 1,96* | 90 |
| VI | 2 % Calciumformiat + 0,8 % Phosphorsäure | 500* | 109 | 1,96* | 90 |
| VII | 2 % Calciumformiat | 490 | 106 | 2,00 | 92 |
| VIII | 0,8 % Phosphorsäure | 450 | 98 | 2,15 | 99 |

* signifikant (p < 0,05)

0 260 391

Beispiel 3:

Verschiedene Zusätze und Kombinationen wurden in einem Broilermastversuch mit insgesamt 560 Tieren in 7 Gruppen von jeweils 80 Tieren geprüft. Der Versuch wurde im Alter von 7 Tagen bis 28 Tagen durchgeführt. In dem Versuch wurde folgende Ration (in Gewichtsprozent) verfüttert:

Mais 46,0
Weizen 10,8
Sojaöl 3,0
Maiskleber 8,0
Sojaextraktionsschrot 24,0
Fischmehl 4,0
Dicalciumphosphat 2,0
Kohlensaurer Kalk 0,9
Vitamine-und Spurenelementmischung 0,8
Aminosäurenvormischung 0,5

Die Gruppe I erhielt diese Normalration ohne irgendwelche Zusätze.

Die Gruppe II erhielt eine entsprechende Ration mit einem Gehalt an 1 Gewichtsprozent Calciumformiat und 1 Gewichtsprozent $NH_4H_2PO_4$. Dafür wurden die Anteile des kohlensauren Kalks um 0,5 Gewichtsprozent, des Weizens um ebenfalls 0,5 Gewichtsprozent und des Dicalciumphosphats um 1 Gewichtsprozent vermindert.

Die Gruppe III erhielt eine entsprechende Ration mit einem Gehalt an 1 Gewichtsprozent Calciumformiat zu Lasten von 0,5 Gewichtsprozent kohlensaurem Kalk und 0,5 Gewichtsprozent Weizen.

Die Gruppe IV erhielt eine entsprechende Ration mit 1 Gewichtsprozent Magnesiumformiat zu Lasten von 1 Gewichtsprozent Weizen und mit 1 Gewichtsprozent $NH_4H_2PO_4$ zu Lasten von 1 Gewichtsprozent Dicalciumphosphat.

Die Gruppe V erhielt eine entsprechende Ration mit 1 Gewichtsprozent Magnesiumformiat zu Lasten von 1 Gewichtsprozent Weizen.

Die Gruppe VI erhielt eine entsprechende Ration mit 1 Gewichtsprozent Fumarsäure zu Lasten von 1 Gewichtsprozent Weizen und mit 1 Gewichtsprozent $NH_4H_2PO_4$ zu Lasten von 1 Gewichtsprozent Dicalciumphosphat.

Die Gruppe VII schließlich erhielt eine entsprechende Ration mit 1 Gewichtsprozent Fumarsäure zu Lasten von 1 Gewichtsprozent Weizen.

In der folgenden Tabelle 3 sind für jede der 7 Gruppen die durchschnittlichen Endgewichte am 28. Lebenstag in Gramm und in %, bezogen auf die Gruppe mit der Normalration ( = 100 %), und die Futterverwertung in g Futter pro g Zunahme und in %, bezogen wiederum auf die Gruppe mit der Normalration ( = 100 %), zusammengestellt:

## Tabelle 3

| Gruppe | | Endgewicht am 28. Lebenstag (g) | (%) | Futterverwertung (g/g) | (%) |
|---|---|---|---|---|---|
| I | Normalration | 618 | 100 | 1,94 | 100 |
| II | Ca-formiat + $NH_4H_2PO_4$ | 660 | 107 | 1,75 | 90 |
| III | Ca-formiat | 654 | 106 | 1,82 | 94 |
| IV | Mg-formiat + $NH_4H_2PO_4$ | 662 | 107 | 1,78 | 92 |
| V | Mg-formiat | 651 | 105 | 1,84 | 95 |
| VI | Fumarsäure + $NH_4H_2PO_4$ | 634 | 103 | 1,90 | 98 |
| VII | Fumarsäure | 630 | 102 | 1,88 | 97 |

Die Ergebnisse zeigen, daß auch bei Geflügel eine deutliche Verbesserung der Leistung durch die erfindungsgemäß zu verwendenden Kombinationen möglich ist.

**Ansprüche**

Verwendung einer Kombination aus Calcium-oder Magnesiumformiat und Phosphorsäure oder einem sauren Salz der Phosphorsäure als Mittel zur Leistungsförderung bei Schweinen und Geflügel.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 751 768 (BASF) <br> * Patentansprüche 1,2; Seite 5, Absatz 4; Seite 9, letzter Absatz * <br> --- | 1 | A 23 K 1/16 <br> A 23 K 1/18 |
| Y | GB-A- 883 927 (AMERICAN CYANAMID COMPANY) <br> * Patentansprüche 1,3; Seite 2, Zeilen 82-89; Beispiele 1-5 * <br> --- | 1 | |
| A | EP-A-0 081 970 (BP CHEMICALS LIMITED) <br> * Patentansprüche 1,3,5 * <br> --- | 1 | |
| A | CHEMICAL ABSTRACTS, Band 32, Nr. 6, März 1938, Spalte 2179(7), Columbus, Ohio, US; W. WÖHLBIER et al.: "Comparative feeding experiments with calcium carbonate and calcium formate with young hens" & BIEDERMANNS ZENTR. B. TIERERNÄHR. 9, 371-3 (1937) <br> * Zusammenfassung * <br> --- | 1 | |
| A | WO-A-8 203 159 (BLICHER, STEEN) <br> * Beispiel 1, Tabellen 1 und 2, Beispiel 2, Tabelle 5 * <br> ----- | 1 | RECHERCHIERTE SACHGEBIETE (Int. Cl.4) <br><br> A 23 K <br> A 61 K |

Der vorliegende Recherchenbericht wurde für alle Patentansprüche erstellt

| Recherchenort | Abschlußdatum der Recherche | Prüfer |
|---|---|---|
| DEN HAAG | 09-12-1987 | DEKEIREL M.J. |